# EUROPEAN PATENT SPECIFICATION

(11) **EP 1 027 887 B1**
(45) Date of publication and mention of the grant of the patent: **13.08.2008**
(21) Application number: 00300546.9
(22) Date of filing: 26.01.2000
(51) Int. Cl.: A61K 9/26

(54) **Matrix controlled release device**
Vorrichtung mit matrixgesteuerter Wirkstofffreisetzung
Dispositif pour la libération du principe actif contrôlée par la matrice

(30) Priority: 10.02.1999 US 119400
(43) Date of publication of application: 16.08.2000
(73) Proprietor: Pfizer Products Inc., Groton, CT 06340 (US)
(72) Inventor: Appel, Leah Elizabeth, Bend, Oregon 97701 (US); Friesen, Dwayne Thomas, Bend, Oregon 97707 (US); Curatolo, William John, Pfizer Inc., Groton, Connecticut 06340 (US); Nightingale, James Alan Schriver, Bend, Oregon 97708 (US); Thombre, Avinash Govind, Pfizer Inc., Groton, Connecticut 06340 (US)
(74) Representative: Kendrick, David Alan

(56) References cited:
- EP-A- 0 344 603
- EP-A- 0 740 934
- EP-A- 0 901 786
- WO-A-93/00889
- WO-A-97/02017
- GIUNCHEDI P ET AL.: "Hydrophilic matrices for the extended release of a model drug exhibiting pH-dependent solubility" INTERNATIONAL JOURNAL OF PHARMACEUTICS,NL,AMSTERDAM, no. 85, 1992, pages 141-147, XP002074951 ISSN: 0378-5173
- YAMAGUCHI T ET AL: "Improvement of Pharmaceutical Properties of 4-O-(4- methoxyphenyl)acetyltylosin Using Solid Dispersion with Carboxymethylethylcellulose" YAKUZAIGAKU,JP,YAKUJI NIPPO-SHA, TOKYO, vol. 53, no. 4, 1993, pages 221-228, XP002121600 ISSN: 0372-7629

## Description

### BACKGROUND OF THE INVENTION

The bioavailability of sparingly water-soluble drugs is well-known to be limited and to be profoundly affected by such factors as the fed state of the patient, the rate of metabolism in relation to the rate of absorption in the gastrointestinal (GI) tract, and the dosage form. Many attempts have been made to improve the form of dosage for such low solubility drugs, generally with a view to attaining an increase in drug concentration, thereby improving the absorption or bioavailability of the drug. Although many of these attempts have been somewhat successful they generally provide an immediate and often temporary increase in absorption in the sense that drug levels in the blood reach an undesirably high level very rapidly. It would be more desirable to simultaneously couple in a single dosage form high bioavailability for sparingly soluble drugs with controlled and sustained release of the drugs. Only a few attempts to accomplish this objective have been reported as methods to improve bioavailability and methods to attain controlled drug release are generally regarded not to be compatible, the conventional wisdom being that use of controlled release techniques would tend to diminish bioavailability.

Exemplary sustained release dosage forms have included crystalline drug particles dispersed in a swellable hydrogel matrix core that releases the drug by diffusion into the environment of use, as described in U.S. Patent No. 4,624,848; a hydrogel reservoir containing a multiplicity of tiny pills wherein each tiny pill consists of a wall surrounding a crystalline drug core, as described in U.S. Patent No. 4,851,232; and a two-layered tablet wherein one layer is crystalline drug mixed with a hydrogel and the other layer is a hydrogel, as described in U.S. Patent No. 5,516,527.

One sustained release dosage form consists of a coated tablet with a core of a solid dispersion of drug in extremely hydrophilic polyoxamer hydrogels that releases the drug by diffusion from the swollen tablet mass and by erosion of the tablet surface, described in PCT Application No. 97/02017. This is in keeping with the widely held view that water-soluble polymers are most suitable for forming solid amorphous dispersions of drugs in polymers. See Ford, 61 Pharm. Acta. Helv. 3 (1986).

U.S. Patents Nos. 4,343,789, 4,404,183 and 4,673,564 all have the same disclosure of a sustained release composition of the vasodilator nicardipine comprising a solid amorphous dispersion of the drug in microcrystalline cellulose, polyethylene oxide, polyvinyl pyrrolidone and the cellulosic polymers hydroxypropylcellulose, hydroxypropylmethylcellulose and hydroxypropylmethylcellulose phthalate. However, the preferred method of forming the dispersion is by extensive and time-consuming ball-milling, and there is no recognition of the concentration-enhancing and amorphous state-stabilizing properties of ionizable cellulosics for forming the drug dispersion.

Solid dispersion dosage forms may be formed by solvent evaporation, by spray drying, by spray coating, by spraying drug solution onto the carrier in a fluidized bed granulator, by twin screw extrusion, by melt fusion, by mechanical admixture such as by ball milling and by mechanical admixture at an elevated but non-melting temperature. See, for example, PCT Application No. 93/11749; European Patent Application No. 0 552 708; U.S. Patent No. 5,456,923; Chowdary et al., 32 Indian Drugs 477 (1995); Dangprasirt et al., 21 Drug Development & Ind. Pharm. 2323 (1995); and Goracinova et al., 22 Drug Development & Ind. Pharm. 255 (1996).

Most solid dispersion drug delivery systems have been directed to the delivery of poorly water-soluble drugs as they generally tend to be immediate release forms; as a result, for drugs with short elimination half-lives, they have those forms' inherent drawbacks of high peak drug concentrations in the blood, short times following administration when drug concentrations in the blood reach a maximum (tₘₐₓ), and relatively short duration of effective levels of concentration in the blood. In addition, although improved bioavailability relative to that for crystalline drug is reported, bioavailability for such dosage forms is nevertheless often low in an absolute sense. Specifically, such drug delivery systems often exhibit little overall improvement in the concentration of drug in a patient's blood over a given time period (commonly referred to as "AUC" in reference to the calculation of the area under a curve comprising a plot of concentration of drug in a patient's blood against time).

In the case of the solid polyoxamer dispersion reported in PCT 97/02017, the dosage form suffers from slow and incomplete release in cases where drug is released by diffusion through a membrane coating due to the inherent low solubility of the drug; conversely, in cases where drug is released by erosion of the drug/polyoxamer dispersion, drug release is typically non-zero order and variable, being dependent on the patient's fed state and gastric retention time. In addition, since the polyoxamer dispersion polymers disclosed are highly hydrophilic and generally require aqueous solvents for dissolution, these polymers cannot be used to form dispersions with hydrophobic drugs via solvent processing as it is difficult or impossible to dissolve the drug and polymer in a common solvent.

Other drug delivery forms are also known in the art. For example the prior art discloses pellets comprising an active principle, a swellable polymeric material and a gastroresistant polymeric material (disclosed in WO 93/00889 and Giunchedi et al, International Journal of Pharmaceutics, 85 (1992) p141-147); and solid pharmaceutical preparations comprising amorphous active compound as a coprecipitate with polyvinylpyrrolidone and a release delaying component (disclosed in EP 0 740 934).

There is still a need in the art for a controlled release dosage form for delivery of a low solubility drug with a short elimination half-life that provides improved drug bioavailability. These needs and others which will become apparent to one skilled in the art are met by the present invention, which is summarized and described in detail below.

### BRIEF SUMMARY OF THE INVENTION

The present invention relates to a controlled release dosage composition comprising:
(a) a solid, substantially homogeneous, dispersion comprising a low solubility drug dispersed in hydroxypropylmethylcellulose acetate succinate cellulosic polymer, a major portion of said drug being amorphous; and
(b) an aqueous - soluble cellulosic polymeric matrix having said dispersion incorporated therein;
wherein the dispersion is formed by spray drying;
wherein a substantially homogenous dispersion is one in which the dispersion itself has a single glass transition temperature (T_{g});
wherein the low solubility drug is one which has a minimum aqueous solubility at physiologically relevant pH 1-8 of 40mg/ml or less; and
wherein a major portion of said drug being amorphous means that at least 60% of the drug in the dispersion is in the amorphous form.

### BRIEF DESCRIPTION OF THE DRAWINGS

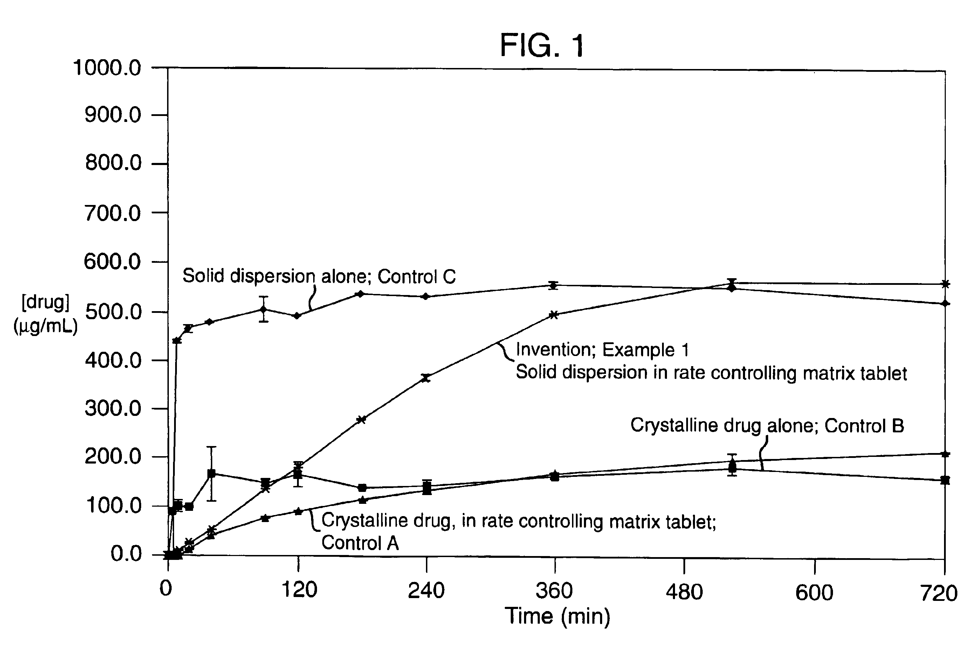

FIGS. 1-4 are graphs comprising plots of release rates of drugs delivered by the controlled release composition of the present invention and of comparative release rates for controls.

### DETAILED DESCRIPTION OF THE INVENTION

The present invention relates to a controlled release dosage composition comprising:
(a) a solid, substantially homogeneous, dispersion comprising a low solubility drug dispersed in hydroxypropylmethylcellulose acetate succinate cellulosic polymer, a major portion of said drug being amorphous; and
(b) an aqueous - soluble cellulosic polymeric matrix having said dispersion incorporated therein;
wherein the dispersion is formed by spray drying;
wherein a substantially homogenous dispersion is one in which the dispersion itself has a single glass transition temperature (T_{g});
wherein the low solubility drug is one which has a minimum aqueous solubility at physiologically relevant pH 1-8 of 40mg/ml or less; and
wherein a major portion of said drug being amorphous means that at least 60% of the drug in the dispersion is in the amorphous form. The term "drug" is conventional, denoting a compound having beneficial, prophylactic, and/or therapeutic properties when administered to an animal, especially to a human. By an "erodible" matrix is meant water-erodible or water-swellable or water-soluble in the sense of being either erodible or swellable or dissolvable in pure water or requiring the presence of an acid or base to ionize the polymeric matrix sufficiently to cause erosion or dissolution. The form of the device may be any known conventional form, including a tablet, a capsule, a caplet, a bead, a multiparticulate, a powder or combinations thereof, and is generally useful in mammals and particularly useful for therapeutic uses in humans.

The drug may be delivered either in the form of gel or a suspension of solids in water or primarily as a solution of the drug, to the extent dissolution has taken place prior to erosion. While not wishing to be bound by any particular theory of delivery mechanism, the delivery is believed to take place by any one or more of the following mechanisms: (1) dissolution of the amorphous drug dispersion in the dosage form prior to erosion, coupled with diffusion from the dosage form, either directly or through a coating; (2) dissolution of the dispersion as the matrix erodes, with delivery primarily as a solution; or (3) delivery as a solid suspension as the matrix erodes, followed by dissolution in the GI tract.

Both the amorphous solid dispersion component and the erodible matrix component may contain osmagens, osmopolymers, solubility-enhancing agents and excipients. In addition, delayed or sustained release features may be added by coating the dosage form with controlled release coating formulations known in the art.

The dosage form of the present invention generally provides controlled delivery of the drug to an environment of use such that (1) the concentration of drug in the in vitro or in vivo environment is enhanced and in turn the bioavailability of the drug is enhanced relative to a comparable dosage form where the drug is present in its undispersed state, *i*.*e*., not incorporated into a solid dispersion before formulation; and (2) the time at which a maximum drug concentration (Cₘₐₓ) in an in vitro or in vivo environment of use is attained is delayed by from 30 minutes to 24 hours.

More specifically, the dosage forms of the invention provide one or more of the following features: (1) they provide a Cₘₐₓ in an aqueous environment in vitro test, which is at least 1.5-fold that achieved by an identical controlled release dosage composition containing the same quantity of drug in an undispersed state; (2) they provide a Cₘₐₓ in an aqueous environment in vitro test, at a time (tₘₐₓ) which is at least 30 minutes longer but not more than 24 hours longer than the tₘₐₓ observed when the solid dispersion is tested without incorporation into a sustained release polymeric matrix; (3) they provide an AUC in drug concentration in an aqueous use environment that is at least 1.25-fold that achieved by an identical controlled release dosage composition containing the same quantity of drug in an undispersed state; (4) when orally dosed to a human or other animal they provide a blood Cₘₐₓ (plasma or serum) that is achieved at a time tₘₐₓ which is at least 30 minutes longer than observed when a control composition is dosed, the control composition comprising the drug dispersion alone, *i*.*e*., not formulated in a sustained release polymeric matrix; (5) when orally dosed to a human or other animal they provide a blood Cₘₐₓ (plasma or serum) that is at least 1.25-fold that observed when a control composition is dosed, the control composition being identical to the test composition with the exception that the drug is formulated in the undispersed state; and (6) when orally dosed to a human or other animal they provide an AUC in drug concentration in the blood that is at least 1.25-fold that observed when a control composition is dosed, the control composition being the same as that described in (5).

### THE DRUG

Prior to formation of the dispersion, the drug in its pure state may be crystalline or amorphous, but when dispersed in the solid dispersion polymer, a major portion of the drug is in an amorphous or non-crystalline state such that its non-crystalline nature is demonstrable by powder X-ray diffraction analysis or by differential scanning calorimetry or by any other standard quantitative measurement. Preferably, the drug is substantially amorphous, and more preferably essentially completely amorphous. As used herein, the term "a major portion" of the drug means that at least 60% of the drug in the dispersion is in the amorphous form, rather than the crystalline form. Preferably, the drug in the dispersion is substantially amorphous. As used herein, "substantially amorphous" means that the amount of the drug in crystalline form does not exceed 25%. More preferably, the drug in the dispersion is essentially completely amorphous, meaning that the amount of drug in crystalline form does not exceed 10% as measured by any of the means noted above. By an "amorphous" state is meant that the drug may be present in (a) discrete drug-rich amorphous domains, (b) homogeneously distributed throughout the dispersion polymer (*e*.*g*., a solid solution), or (c) any state or combination of states between the extremes of (a) and (b).

The solid dispersion is preferably substantially homogeneous so that the drug is dispersed as homogeneously as possible throughout the polymer. As used herein, "substantially homogeneous" means that the drug present in relatively pure amorphous domains within the solid dispersion is relatively small, on the order of less than 20%, and preferably less than 10%. While the dispersion may have some drug-rich domains, it has a single glass transition temperature (T_{g}) which demonstrates that the dispersion is substantially homogenous. This contrasts with a physical mixture of pure amorphous drug particles and pure amorphous polymer particles which generally display two distinct T_{g}s, one that of the drug and one that of the polymer.

Since the solubility of a given drug is often pH-dependent, the device of the present invention is appropriate for delivery of any drug the solubility of which, over any portion of the physiologically relevant pH range, falls into the solubility ranges noted herein. In general, the class of drugs may be characterized by having a solubility sufficiently low that it is desirable to increase the drug's solubility either (a) within the dosage form to improve its delivery characteristics or (b) outside the dosage form to improve the rate or extent of drug absorption.

The drug is a "low-solubility drug," meaning that the drug has a minimum aqueous solubility at a physiologically relevant pH (*e*.*g*., pH 1-8) of 40 mg/ml or less. Thus, the drug may be either "substantially water-insoluble," that is having a minimum aqueous solubility at a physiologically relevant pH of less than 0.01 mg/mL, or "sparingly water-soluble," that is, having a water solubility up to 1 to 2 mg/mL, or even moderate solubility where the solubility is as high as 20 to 40 mg/mL. In general, it may be said that the drug has a dose-to-aqueous solubility ratio greater than 5 mL, where the drug solubility is the minimum value observed in any physiologically relevant aqueous solution including USP simulated gastric and intestinal buffers.

In some cases, it is also desirable to enhance the solubility of the drug within the dosage form to increase the rate of diffusion or release from the dosage form or to improve the absorption of drug in the colon. In such cases, the invention may be applied to drugs with a minimum aqueous solubility as high as 20 to 40 mg/mL. This is particularly true when it is desired to deliver a solution of the drug. In such cases, the dose-to-aqueous solubility ratio may be as low as 1 mL.

Virtually any beneficial therapeutic agent that meets the solubility criteria may be used as the drug in the present invention. In addition, the drug may be employed in the form of its pharmaceutically acceptable salts as well as in anhydrous and hydrated forms and prodrugs. Preferred classes of drugs include, but are not limited to, antihypertensives, antidepressants, antianxiety agents, anti-atherosclerotic agents, anticlotting agents, anticonvulsants, blood glucose-lowering agents, decongestants, antihistamines, antitussives, anti-inflammatories, antipsychotic agents, cognitive enhancers, cholesterol-reducing agents, antiobesity agents, autoimmune disorders agents, anti-impotence agents, antibacterial and antifungal agents, hypnotic agents, anti-Parkinsonism agents, antibiotics, antiviral agents, anti-impotence agents, anti-neoplastics, barbituates, sedatives, nutritional agents, beta blockers, emetics, anti-emetics, diuretics, anticoagulants, cardiotonics, androgens, corticoids, anabolic agents, anti-depression agents, anti-infective agents, coronary vasodilators, carbonic anhydrase inhibitors, antiprotozoals, gastrointestinal agents, serotonin antagonists, anesthetics, hypoglycemic agents, dopaminergic agents, anti-Alzheimer's Disease agents, anti-ulcer agents, platelet inhibitors, and glycogen phosphorylase inhibitors.

Specific examples of the above and other classes of drugs and therapeutic agents deliverable by the invention are set forth below, by way of example only. Specific examples of antihypertensives include prazosin, nifedipine, trimazosin and doxazosin mesylate; a specific example of an antianxiety agent is hydroxyzine; a specific example of a blood glucose-lowering agent is glipizide; a specific example of an anti-impotence agent is sildenafil citrate; specific examples of anti-neoplastics include chlorambucil, lomustine and echinomycin; specific examples of anti-inflammatory agents include betamethasone, prednisolone, aspirin, flurbiprofen and (+)-N-{4-[3-(4-fluorophenoxy)phenoxy]-2-cyclopenten-1-yl}-N-hyroxyurea; a specific example of a barbiturate is phenobarbital; specific examples of antivirals include acyclovir and virazole; specific examples of vitamins/nutritional agents include retinol and vitamin E; specific examples of a β-blocker include timolol and nadolol; a specific example of an emetic is apomorphine; specific examples of a diuretic include chlorthalidone and spironolactone; a specific example of an anticoagulant is dicumarol; specific examples of cardiotonic include digoxin and digitoxin; specific examples of an androgen include 17-methyltestosterone and testosterone; a specific example of a mineral corticoid is desoxycorticosterone; a specific example of a steroidal hypnotic/anesthetic is alfaxalone; specific examples of an anabolic agent include fluoxymesterone and methanstenolone; specific examples of antidepression agents include fluoxetine, paroxitine, venlafaxine, sertraline, sulpiride, [3,6-dimethyl-2-(2,4,6-trimethyl-phenoxy)-pyridin-4-yl]-(l-ethylpropyl)-amine and 3,5-dimethyl-4-(3'-pentoxy)-2-(2',4',6'-trimethylphenoxy)pyridine; specific examples of an antibiotic include ampicillin and penicillin G; specific examples of an anti-infective include benzalkonium chloride and chlorhexidine; specific examples of a coronary vasodilator include nitroglycerin and mioflazine; a specific example of a hypnotic is etomidate; specific examples of a carbonic anhydrase inhibitor include acetazolamide and chlorzolamide; specific examples of an antifungal include econazole, terconazole and griseofulvin; a specific example of an antiprotozoal is metronidazole; a specific example of an imidazole-type anti-neoplastic is tubulazole; specific examples of an anthelmintic agent include thiabendazole and oxfendazole; specific examples of an antihistaminic include astemizole, levocabastine and cinnarizine; specific examples of antipsychotics include fluspirilene, penfluridole and ziprasidone; specific examples of a gastrointestinal agent include loperamide and cisapride; specific examples of a serotonin antagonist include ketanserin and mianserin; a specific example of an anesthetic is lidocaine; a specific example of a hypoglycemic agent is acetohexamide; a specific example of an anti-emetic is dimenhydrinate; a specific example of an antibacterial is cotrimoxazole; a specific example of a dopaminergic agent is L-DOPA; specific examples of anti-Alzheimer agents are THA and donepezil; a specific example of an anti-ulcer agent/H2 antagonist is famotidine; specific examples of a sedative/hypnotic include chlordiazepoxide and triazolam; a specific example of a vasodilator is alprostadil; a specific example of a platelet inhibitor is prostacyclin; specific examples of an ACE inhibitor/antihypertensive include enalaprilic acid and lisinopril; specific examples of a tetracycline antibiotic include oxytetracycline and minocycline; specific examples of a macrolide antibiotic include azithromycin, clarithromycin, erythromycin and spriamycin; specific examples of glycogen phosphorylase inhibitors include [R-(R*S*)]-5-Chloro-N-[2-hydroxy-3-[methoxymethylamino}-3-oxo-l-(phenylmethyl)-propyl]propyl]-1H-indole-2-carboxamide and 5-chloro-1H-indole-2-carboxylic acid [(lS)-benzyl-3((3R,4S)-dihydroxypyrrolidin-1-yl-)-(2R)-hydroxy-3-oxypropyl]amide.

Further examples of drugs deliverable by the invention are the glucose-lowering drug chlorpropamide, the anti-fungal fluconazole, the anti-hypercholesterodemic atorvastatin calcium, the antipsychotic thiothixene hydrochloride, the anxiolytics hydroxyzine hydrochloride and doxepin hydrochloride, the anti-hypertensive amlodipine besylate, the anti-inflammatories iroxicam, valdecoxib and celicoxib, and the antibiotics carbenicillin indanyl sodium, bacampicillin hydrochloride, troleandomycin, and doxycycline hyclate.

### THE DISPERSION POLYMER

The drug is dispersed in hydroxypropymethyl cellulose acetate succinate polymer. Suitable polymers for forming the solid dispersion of drug are preferably polymeric, concentration-enhancing, non-aqueous solvent-processable, non-toxic and inert. By "non-aqueous solvent" is meant solvents comprising up to about 30 wt% water.

By "non-aqueous solvent-processable" is meant the polymer is capable of being processed with the drug in a common non-aqueous solvent to form the solid dispersion, *i*.*e*., it is generally adaptable to techniques utilizing non-aqueous solvents in the formation of solid dispersions. Such techniques include spray-drying or spray coating. The polymer has a preferred solubility in the non-aqueous solvent of at least 0.1 mg/mL, more preferably greater than 1 mg/mL and most preferably greater than 10 mg/mL. This property is critical for forming the solid amorphous dispersion of the drug and dispersion polymer via solvent processing as the drug and dispersion polymer must be dissolved in a common solvent and such solvents cannot be substantially aqueous as the drugs by definition have a relatively low aqueous solubility.

Such dispersion polymers are aqueous-soluble in the sense that they are sufficiently soluble (≥1 mg/mL) in at least a portion of the 1 to 8 pH range that they exhibit a "concentration-enhancing" property. By "concentration-enhancing" is meant the concentration of drug provided by a solid dispersion of the drug in aqueous media is at least 1.5-fold that provided by an equivalent quantity of undispersed drug.

By "non-toxic" is meant that they should be acceptable for oral administration to a mammal, especially a human.

By "inert" is meant not adversely reactive or bioactive, yet still capable of positively affecting the drug's bioavailability.

The amount of the concentration-enhancing dispersion polymer present in the dispersion generally ranges from about 10 to about 95 wt%, preferably 30 to 80 wt%.

It should be noted that in the above polymer nomenclature, ether-linked substituents are recited prior to "cellulose" as the moeity attached to the ether group (e.g., "ethylbenzoic acid cellulose" has ethoxybenzoic acid substituents) and ester-linked substituents are recited after "cellulose" as the carboxylate (*e*.*g*., "cellulose phthalate" has one carboxylic acid of each phthalate moiety ester-linked to the polymer and the other carboxylic acid unreacted).

It should further be noted that a polymer name such as "cellulose acetate phthalate" refers to any of the family of cellulosic polymers that have acetate and phthalate groups attached via ester linkages to a significant fraction of the cellulosic polymer's hydroxyl groups. Generally, the degree of substitution of each substituent group can range from 0.1 to 2.8 as long as the other criteria of the polymer are met. "Degree of substitution" refers to the average number of the three hydroxyls per saccharide repeat unit on the cellulose chain that have been substituted. For example, if all of the hydroxyls on the cellulose have been phthalate-substituted, the phthalate degree of substitution is 3. Also included within each polymer family type are cellulosic polymers that have additional substituents added in relatively small amounts that do not substantially alter the performance of the polymer.

### THE SOLID AMORPHOUS DISPERSION

The solid dispersion generally contains from 5 to 90 wt% drug, preferably 20 to 70 wt%. However, when the drug dose is less than 25 mg, the drug content of the dispersion may be less than 5 wt%.

The solid amorphous dispersion of drug is prepared by spray-drying.

This method of forming the dispersion is by dissolving the drug and matrix polymer in a common solvent, then removing the solvent by spray-drying or spray-coating the mixture. Spray-drying and spray-coating processes and equipment are described generally in Perry's Chemical Engineer's Handbook, pages 20-54 to 20-57 (6th Ed. 1984). More details on spray-drying processes and equipment are reviewed by Marshal in 50 Chem. Eng. Prog. Monogr. Series 2 (1954).

The terms "spray-drying" and "spray-coating" in connection with the present invention are used conventionally and broadly refer to processes involving breaking up liquid mixtures into small droplets (atomization) and rapidly removing solvent from the mixtures in a vessel such as a spray-drying apparatus, fluidized bed- or pan-coater where there is a strong driving force for evaporation of solvent from the droplets. In the case of spray-coating the droplets impinge on a particle, bead, pill, tablet, or capsule, resulting in a coating comprising the solid amorphous dispersion. Spray-coating may also be conducted on a metal, glass or plastic surface and the coated layer may subsequently be removed and milled to the desired particle size. In the case of spray-drying, the droplets generally dry prior to impinging on a surface, thus forming particles of solid amorphous dispersion on the order of 1 to 100 µm in diameter. The strong driving force for solvent evaporation is generally provided by maintaining the partial pressure of solvent in the spray-drying apparatus well below the vapor pressure of the solvent at the temperature of the drying droplets. This is accomplished by either (1) maintaining the pressure in the spray-drying apparatus at a partial-vacuum (*e*.*g*., 0.01 to 0.50 atm); (2) mixing the liquid droplets with a warm drying gas; or (3) both. For example, a solution of drug and a dispersion polymer such as HPMCAS in acetone may be suitably spray-dried by spraying the solution at a temperature of 50°C (the vapor pressure of acetone at 50°C is about 0.8 atm) into a chamber held at 0.01 to 0.2 atm total pressure by connecting the outlet to a vacuum pump. Alternatively, such a solution may be sprayed into a chamber where it is mixed with nitrogen gas at a temperature of 80°C to 250°C and a pressure of 1.0 to 1.2 atm.

Generally, the temperature and flow rate of the drying gas is chosen so that dispersion polymer/drug solution droplets are dry enough by the time they reach the wall of the apparatus that they are essentially solid, so that they form a fine powder and do not stick to the apparatus wall. The actual length of time to achieve this level of dryness depends on the size of the droplets. Droplet sizes generally are larger than about 1 µm in diameter, with 5 to 100 µm being typical. The large surface-to-volume ratio of the droplets and the large driving force for evaporation of solvent leads to actual drying times of a few seconds or less. For some mixtures of drug/dispersion polymer/solvent this rapid drying is critical to the formation of a uniform, homogeneous composition and generally preventing the mixture from separating into drug-rich and polymer-rich phases, although a limited degree of phase separation is permissible. Such dispersions having a homogenous composition can be considered solid solutions and may be supersaturated in drug. Such homogeneous dispersions are preferred in that the maximum drug concentration (MDC) value obtained when a large amount of drug is dosed can be higher for such dispersions relative to dispersions for which at least a portion of the drug is present as a drug-rich amorphous or crystalline phase.

Solidification times should be less than 100 seconds, preferably less than a few seconds, and more preferably less than 1 second. In general, to achieve such rapid solidification of the drug/polymer solution, it is preferred that the diameter of droplets formed during the spray-drying process are less than 100 µm, preferably less than 50 µm, and most preferably less than 25 µm. The so-formed solid particles resulting from solidification of these droplets generally tend to be 2 to 40 µm in diameter.

Following solidification, the solid powder typically remains in the spray-drying chamber for 5 to 60 seconds, evaporating more solvent. The final solvent content of the solid dispersion as it exits the dryer should be low, since low solvent content tends to reduce the mobility of drug molecules in the dispersion, thereby improving its stability. Generally, the residual solvent content of the dispersion should be less than 10 wt% and preferably less than 2 wt%.

The solution spray-dried to form the polymer/drug dispersion can be quite simple, containing only drug and polymer in a solvent. Generally, the ratio of polymer to drug in the solution ranges from 0.1 to 20 and preferably ranges from 0.5 to 5. However, when the drug dose is low (less than 20 mg), the polymer to drug ratio may be even greater than 5.

Other excipients may be added to the spray solution, either co-dissolved in the solvent along with the drug and dispersion polymer or suspended in the solution to form a slurry. Such excipients may include: acids, bases or buffers to modify the ionic state and dissolution properties of the resulting dispersion; fillers, binders, disintegrants or other materials to improve the tableting process or final properties of the dosage form; antioxidants to improve the dispersions stability; osmotic agents, including both water-swellable hydrophilic polymers and osmogens such as hygroscopic sugars, organic acids and polyols; and surfactants to affect the wetting of the dosage form.

Solvents suitable for spray-drying may generally be characterized as "non-aqueous" in the same sense mentioned above, *i.e.*, comprising <30 wt% water. They may be essentially any organic compound in which the drug and polymer are mutually soluble or mixtures of water and/or organic compounds. In the case of mixtures of water and organic compounds, up to about 30 wt% water may advantageously be included, particularly in those cases where the organic solvent is more hydrophobic than the drug. Preferably, the solvent is also relatively volatile with a boiling point of 150°C or less. Although lower volatility solvents may also be used, they are generally less preferred. In those cases where the solubility of the drug in the volatile solvent is low, it may be desirable to include a small amount, say 2 to 25 wt%, of a low volatility solvent such as N-methylpyrrolidone (NMP) or dimethylsulfoxide (DMSO) in order to enhance drug solubility. In addition, the solvent should have relatively low toxicity and be removed from the dispersion to a level that is acceptable according to the International Committee on Harmonization (ICH) guidelines. Removal of solvent to this level may require a processing step such as spray-drying subsequent to the spray-drying or spray-coating dispersion formulation process.

Preferred solvents include alcohols such as methanol, ethanol, n-propanol, isopropanol, and butanol; ketones such as acetone, methyl ethyl ketone and methyl isobutyl ketone; esters such as ethyl acetate and propylacetate; and various other solvents such as acetonitrile, methylene chloride, toluene, and 1,1,1-trichloroethane. Lower volatility solvents such as dimethyl acetamide or DMSO may also be used. Mixtures of solvents may also be used, as may mixtures up to 30 wt% with water as long as the polymer and drug are sufficiently soluble to make the spray-drying process practical.

In general, the solid amorphous dispersion provides a cₘₐₓ in a use environment that is at least 1.25-fold that of a control dosage form comprising an equivalent amount of undispersed drug (typically the control is simply the crystalline drug alone in its thermodynamically most stable form unless the crystalline form of the drug is unknown, in which case the control is the amorphous drug alone).

Alternatively, the dispersion of the present invention when tested *in vitro* in a physiologically relevant aqueous solution provides an AUC value at least 1.5-fold that measured for an equivalent quantity of undispersed drug. Preferably, when orally administered, the dispersion also provides an AUC *in vivo* (area under the blood drug concentration vs. time plot) that is at least 1.25-fold that observed when an equivalent quantity of undispersed drug is dosed.

### OTHER DISPERSION COMPONENTS

The solid amorphous drug dispersion of the drug delivery device may contain a wide variety of additives and excipients, generally aimed at enhancing the bioavailability of the drug.

The dispersion may include osmotically effective solutes, often referred to as "osmogens." Typical useful osmogens include magnesium sulfate, magnesium chloride, calcium chloride, sodium chloride, lithium chloride, potassium sulfate, sodium carbonate, sodium sulfite, lithium sulfate, potassium chloride, sodium sulfate, d-mannitol, urea, sorbitol, inositol, raffinose, sucrose, glucose, fructose and mixtures thereof. Particularly preferred osmagens are glucose, lactose, sucrose, sodium chloride, mannitol and xylitol.

The solid dispersion may also include 1 to 20 wt% solubility-enhancing or -reducing agents that promote or retard the dissolution rate of the dispersion, including the drug. Examples of suitable solubility-enhancing agents include surfactants; pH control agents such as buffers, organic acids and organic acid salts and organic and inorganic bases; glycerides; partial glycerides; glyceride derivatives; polyhydric alcohol esters; PEG and PPG esters; sorbitan esters; polyoxyethylene sorbitan esters; carbonate salts; alkyl sulfonates; and cyclodextrins.

Exemplary solubility-enhancing or -reducing agents are: organic acids such as citric acid; organic acid salts such as calcium acetate; partial glycerides such as glyceryl monostearate; glycerides such as triacetin; glyceride derivatives such as glyceryl succinate; polyethylene glycol esters such as polyoxyethylene laurate; polypropylene glycol esters such as polyoxypropylene stearate; polyhydric alcohol esters such as glucose laurate; sorbitan esters such as polysorbate 80; carbonate salts such as calcium carbonate; alkyl sulfonates such as sodium lauryl sulfate; and cyclodextrins such as sulphobutylether betacyclodextrin.

The solid dispersion may include additives or excipients that promote stability, tableting or processing of the dispersion. Such components include tableting aids, surfactants, water-soluble polymers, pH modifiers, fillers, binders, pigments, disintegrants, lubricants and flavorants. Exemplary of such components are microcrystalline cellulose; metallic salts of acids such as aluminum stearate, calcium stearate, magnesium stearate, sodium stearate, and zinc stearate; fatty acids, hydrocarbons and fatty alcohols such as stearic acid, palmitic acid, liquid paraffin, stearyl alcohol, and palmitol; fatty acid esters such as glyceryl (mono-and di-) stearates, triglycerides, glyceryl (palmiticstearic) ester, sorbitan monostearate, saccharose monostearate, saccharose monopalmitate, and sodium stearyl fumarate; alkyl sulfates such as sodium lauryl sulfate and magnesium lauryl sulfate; and inorganic materials such as talc and dicalcium phosphate.

All such additives and excipients may be added directly to the spray-drying solution such that the additive is dissolved or suspended in the solution as a slurry. Alternatively, such components may be added following the spray-drying process to aid in forming the final dosage form.

### THE ERODIBLE MATRIX

The composition also comprises an aqueous-soluble cellulosic polymer matrix into which said dispersion is incorporated. The erodible polymeric matrix into which the solid dispersion is incorporated may generally be described as a set of excipients that are mixed with the dispersion following its formation that, when contacted with the aqueous environment of use imbibes water and forms a water-swollen gel or "matrix" that entraps the dispersion. Drug release may occur by a variety of mechanisms: the matrix may disintegrate or dissolve from around the dispersion particles or granules; or the drug may dissolve in the imbibed aqueous solution and diffuse from the tablet, beads or granules of the dosage form. A key ingredient of this water-swollen matrix is the water-swellable, erodible, or soluble polymer which may generally be described as a osmopolymer, hydrogel or water-swellable polymer. Such polymers may be linear, branched, or crosslinked. They may be homopolymers or copolymers.

The class of cellulosics for the erodible matrix comprises aqueous-soluble and aqueous-erodible cellulosics such as ethyl cellulose (EC), methylethyl cellulose (MEC), CMC, CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, HPMC, HPMCP, HPMCAS, HPMCAT, and ethylhydroxy ethylcellulose (EHEC). A particularly preferred class of such cellulosics comprises various grades of low viscosity (MW ≤50,000 daltons) and high viscosity (MW ≥50,000 daltons) HPMC. Commercially available low viscosity HPMC polymers include the Dow METHOCEL series E5, E15LV, E50LV and K100LY, while high viscosity HPMC polymers include E4MCR, E10MCR, K4M, K15M and K100M; especially preferred in this group are the METHOCEL^{™} K series. Other commercially available types of HPMC include the Shinetsu METOLOSE 90SH series.

Although the primary role of the erodible matrix material is to control the rate of release of drug to the environment of use, the inventors have found that the choice of matrix material can have a large effect on the maximum drug concentration attained by the controlled-release dosage form as well as the maintenance of a high drug concentration. The proper choice of polymer in turn affects the bioavailability of the drug. We have found that water-soluble cellulosics such as certain grades of methyl cellulose (MC) or HPMC, when used as the primary rate-controlling matrix material, can result in higher maximum drug concentrations *in vitro* relative to other conventional matrix polymers such as polyoxamers (*e*.*g*., PEO or PEG) or carboxylic acid polymers such as CMC or calcium CMC or polyacrylic acids such as Carbopol. Thus, an especially preferred embodiment of the invention comprises a solid substantially amorphous dispersion of drug in a cellulosic polymer incorporated into controlled-release beads, granules, or tablets wherein the matrix polymer comprises an aqueous-soluble cellulosic. It has been found that in an aqueous use environment such dosage forms provide an area under the drug concentration versus time plot that is at least 1.25-fold that of an identical control dosage composition except that the polymeric matrix comprises PEO, wherein the PEO has a molecular weight such that the time to release 50% of the drug from the control is greater than 80% but less than 120% the time to release 50% of the drug from the dosage composition of the present invention. Exemplary of such cellulosics are MC, HEC, HPC, hydroxyethylmethyl cellulose, HPMC, and other closely related water-soluble polymers. Preferably, the matrix material comprises MC or HPMC.

Other materials useful as the erodible matrix material include, but are not limited to, pullulan, polyvinyl pyrrolidone, polyvinyl alcohol, polyvinyl acetate, glycerol fatty acid esters, polyacrylamide, polyacrylic acid, copolymers of ethacrylic acid or methacrylic acid (EUDRAGIT^{®}, Rohm America, Inc., Piscataway, New Jersey) and other acrylic acid derivatives such as homopolymers and copolymers of butylmethacrylate, methylmethacrylate, ethylmethacrylate, ethylacrylate, (2-dimethylaminoethyl)methacrylate, and (trimethylaminoethyl) methacrylate chloride.

The erodible matrix polymer may contain concentration-enhancing dispersion polymers of the type discussed above. In addition, the erodible matrix polymer may contain a wide variety of the same types of additives and excipients known in the pharmaceutical arts and discussed above, including osmopolymers, osmagens, solubility-enhancing or -retarding agents and excipients that promote stability or processing of the dosage form.

### ENTERIC COATINGS

The dosage compositions of the present invention may also be overcoated with one or more pH-sensitive coating compositions, commonly referred to in the art as "enteric coatings," according to conventional procedures in order to delay the release of drug. Suitable pH-sensitive polymers include those which are relatively insoluble and impermeable at the pH of the stomach, but which are more soluble or disintegrable or permeable at the pH of the small intestine and colon. Such pH-sensitive polymers include polyacrylamides, phthalate derivatives such as acid phthalate of carbohydrates, amylose acetate phthalate, cellulose acetate phthalate (CAP), other cellulose ester phthalates, cellulose ether phthalates, hydroxypropylcellulose phthalate (HPCP), hydroxypropylethylcellulose phthalate (HPECP), hydroxyproplymethylcellulose phthalate (HPMCP), HPMCAS, methylcellulose phthalate (MCP), polyvinyl acetate phthalate (PVAcP), polyvinyl acetate hydrogen phthalate, sodium CAP, starch acid phthalate, cellulose acetate trimellitate (CAT), styrene-maleic acid dibutyl phthalate copolymer, styrene-maleic acid/polyvinylacetate phthalate copolymer, styrene and maleic acid copolymers, polyacrylic acid derivatives such as acrylic acid and acrylic ester copolymers, polymethacrylic acid and esters thereof, polyacrylic and methacrylic acid copolymers, shellac, and vinyl acetate and crotonic acid copolymers.

Preferred pH-sensitive polymers include shellac; phthalate derivatives; CAT; HPMCAS, polyacrylic acid derivatives, particularly copolymers comprising acrylic acid and at least one acrylic acid ester; polymethyl methacrylate blended with acrylic acid and acrylic ester copolymers; and vinyl acetate and crotonic acid copolymers.

A particularly preferred group of pH-sensitive polymers includes CAP, PVAcP, HPMCP, HPMCAS, anionic acrylic copolymers of methacrylic acid and methylmethacrylate, and osmopolymers comprising acrylic acid and at least one acrylic acid ester.

Cellulose acetate phthalate may be applied as an enteric coating to the dosage forms of the invention to provide delayed release of drug until the dosage form has exited the stomach. The CAP coating solution may also contain one or more plasticizers, such as diethyl phthalate, polyethyleneglycol-400, triacetin, triacetin citrate, propylene glycol, and others as known in the art. Preferred plasticizers are diethyl phthalate and triacetin. The CAP coating formulation may also contain one or more emulsifiers, such as polysorbate-80.

Anionic acrylic copolymers of methacrylic acid and methylmethacrylate are also particularly useful enteric coating materials for delaying the release of drug until the tablets have moved to a position in the GI tract which is distal to the stomach. Copolymers of this type are available from Rohm America, Inc., under the trade names EUDRAGIT-L^{®} and EUDRAGIT-S^{®}. EUDRAGIT-L^{®} and EUDRAGIT-S^{®} are anionic copolymers of methacrylic acid and methylmethacrylate. The ratio of free carboxyl groups to the esters is approximately 1:1 in EUDRAGIT-L^{®} and approximately 1:2 in EUDRAGIT-S^{®}. Mixtures of EUDRAGIT-L^{®} and EUDRAGIT-S^{®} may also be used. For coating these acrylic coating polymers can be dissolved in an organic solvent or mixture of organic solvents or suspended in aqueous media. Useful solvents for this purpose are acetone, isopropyl alcohol, and methylene chloride. It is generally advisable to include 5-20 wt% plasticizer in coating formulations of acrylic copolymers. Useful plasticizers include polyethylene glycols, propylene glycols, diethyl phthalate, dibutyl phthalate, castor oil, and triacetin. EUDRAGIT-L^{®} is preferred because it dissolves relatively quickly at intestinal pH.

In addition to the pH-sensitive polymers listed above, delayed release coatings may consist of a mixture or blend of two or more pH-sensitive polymers or may consist of a mixture of one or more pH-sensitive polymers and one or more non-pH-sensitive polymers. Addition of a non-pH-sensitive polymer to the pH-sensitive polymer is useful in modulating the duration of the delay or rate of release of drug from the granule, bead or tablet. For example, the delay can be lengthened by blending an aqueous-insoluble polymer with the pH-sensitive polymers, while the delay can be shortened by blending a water-soluble polymer with the pH-sensitive polymers. Preferred non-pH-sensitive aqueous-insoluble polymers include cellulose esters, cellulose ethers, polyacrylates, polyamides, polyesters, and vinyl polymers. Preferred non-pH-sensitive aqueous-soluble polymers include hydroxyalkyl-substituted cellulosics such as HPC, HEC and HPMC, PVA, PEG, PEO, PEG/PPG copolymers, and aqueous-soluble polyamides, polysaccharides, and polyacrylates. Various additives may be included in such coatings, including emulsifiers, plasticizers, surfactants, fillers and buffers.

Finally, the polymeric coating may be described as being "quasi-enteric" in the sense that it remains substantially intact for a significant period of time (e.g., greater than an hour) after the dosage form exits the stomach, thereafter becoming sufficiently drug-permeable to permit gradual release of drug by diffusion through the coating.

### USE AND FABRICATION

In use, the solid dispersion in the erodible matrix absorbs water from the environment of use to form a water-swollen mass. Drug may be released by diffusion from the dispersion or by the dispersion slowly eroding into the environment of use. Thus drug is released either primarily as a solution of drug or as a suspension of drug; when delivered as a suspension, the drug formulation subsequently dissolves in the environment of use, such as the GI tract.

Dosage forms of the present invention may be made by essentially any known process suitable for fabrication of pharmaceutical tablets, caplets, capsules, beads, multiparticulates, powders for suspensions or unit dosage packets, commonly referred to as sachets, wherein the solid dispersion is substituted for crystalline drug. For example, the ingredients may be blended, milled and wet- or dry-granulated to form a homogeneous blend of ingredients. Blended ingredients may be compressed into tablets using conventional tablet presses or ingredients may be segregated and then compressed to form tablets with a layered or coated geometry. Additionally, when the dosage form comprises multiparticulates, beads or powders, such materials are prepared, for example, by melt-congealing from a spinning disc, extrusion-spheronization, or fluid-bed granulation, or by coating nonpareil seeds with a mixture of water or organic solvent and the dosage form components.

Following formation of the solid drug dispersion and erodible polymeric matrix in tablet, caplet, bead, powder, multiparticulate or capsule form, it may additionally be coated with a pH-sensitive enteric or quasi-enteric coating, or with a coating to mask taste, improve appearance, or facilitate swallowing of the dosage form. Such coatings may be fabricated by any conventional means including fluidized bed coating, spray-coating, pan-coating and powder-coating using aqueous or organic solvents. Additionally, the dosage form may comprise an immediate release layer of the same or different drug from that used to form the solid dispersion, the drug(s) being in crystalline, amorphous or dispersion form.

The dosage forms of this invention are useful in treating a variety of conditions and diseases, including those exemplified herein, by administering the dosage forms described herein to a mammal in need of such treatment.

### EXAMPLE 1

Exemplary dosage forms of the present invention were fabricated by first forming a batch of 1:2 solid dispersion ("SD") comprising 1 part of a low solubility drug to 2 parts of dispersion polymer by mixing the drug 5-chloro-1H-indole-2-carboxylic acid [(1S) -benzyl-3-((3R,4S) -dihydroxypyrrolidin-1-yl-) - (2R) -hydroxy-3-oxypropyl]amide (a glycogen phosphorylase inhibitor from Pfizer, Inc.) having a water solubility of 80 µg/mL, in the solvent acetone together with a "medium fine" (MF) grade of HPMCAS (AQUOT, Shinetsu, Tokyo, Japan) to form a solution. The makeup of the solution was 2.5 wt% drug, 5 wt% polymer and 92.5 wt% solvent. This solution was then spray-dried by directing an atomizing spray via a Niro two-fluid nozzle at 2.8 bar and 200 g/min feed rate into a stainless steel chamber of a Niro portable spray-dryer maintained at 180°C at the inlet and 70°C at the outlet. Portions of the drug dispersion were held back and subjected to analysis by imaging the dispersion using scanning electron microscopy, and so verified to be in an amorphous, non-crystalline state.

To incorporate the resulting SD into the erodible polymeric matrix, 1.05 g of the SD particles were then mixed with 1.7 g HPMC (METHOCEL K 100 LV prem., Dow Chemical, Midland, Michigan), 0.70 g of the lactose filler FAST FLOW (Foremost/Van Water and Rogers, Baraboo, Wisconsin), and 0.0525 g of the lubricant magnesium stearate, all blended for 20 minutes in a TURBULA blender (Willy A. Bachofen AG Muschinenfabrik, Basel, Switzerland) to render the mixture homogeneous. The so-formed homogeneous core mixture contained 10 wt% drug, 20 wt% HPMCAS-MF, 48.5% METHOCEL, 20 wt% lactose and 1.5 wt% magnesium stearate. This homogeneous mixture was formed into tablets using an F-3 Press (Manesty, Liverpool, England) with 11/32" tooling. The tablet weight was about 350 mg.

As a control (Control A), tablets were similarly prepared except rather than using the drug/HPMCAS-MF SD, an undispersed mixture of 1 part crystalline drug to 2 parts dispersion polymer (HPMCAS-MF) was used. As a second control (Control B), 37.4 mg of crystalline drug with no erodible matrix was used. As a third control (Control C), 106.1 mg of the SD with no erodible matrix polymer was used.

Phosphate buffered saline solution (PBS) was prepared comprising 20 mM sodium phosphate, 466 mM potassium phosphate, 87 mM NaCl and 0.2 mM KCl, adjusted to pH 6.5. Four *in vitro* dissolution tests were performed in 35 mL of the PBS solution at 37°C to test the effectiveness of drug release from the dosage composition of the invention as measured against Controls A, B and C. Drug concentrations over time were determined by periodically withdrawing samples of each of the four solutions, centrifuging the samples for 1 minute at 13,000 rpm to pellet any undissolved drug, sampling the supernate, and analyzing the sample by High Performance Liquid Chromatography (HPLC) to thereby calculate drug concentrations.

The results are set forth in Table 1 and graphically illustrated in Figure 1. As is apparent from Table 1 and Figure 1, the dosage form of the present invention exhibited a gradual and continuous increase in drug concentration in the PBS solution over a period of about 8 hours, reaching a maximum value of about 563 µg/mL, or about three-fold that achieved by either Control A or Control B. Finally, the data obtained demonstrate that supersaturated drug concentrations are achievable by the present invention. The matrix tablets were completely disintegrated over the course of the 12 hour dissolution tests, indicating that the mechanism of controlled release was primarily by erosion.

**Table 1**

| Dosage Form | Time (min) | [Drug]* | AUC** |
|---|---|---|---|
| Example 1 of | 0 | 0.0 | 0 |
| invention; SD | 4 | 0.0 | 0 |
| in erodible matrix | 10 | 11.2 | 34 |
| | 20 | 25.3 | 217 |
| | 40 | 52.9 | 999 |
| | 90 | 136.3 | 5730 |
| | 120 | 178.3 | 10449 |
| | 180 | 277.7 | 24131 |
| | 240 | 365.6 | 43432 |
| | 360 | 496.8 | 95177 |
| | 525 | 563.2 | 182625 |
| | 720 | 562.9 | 292415 |
| Control A; | 0 | 0.0 | 0 |
| Crystalline drug | 4 | 0.0 | 0 |
| mixed with | 10 | 0.0 | 0 |
| dispersion polymer | 20 | 13.2 | 66 |
| | 40 | 41.7 | 614 |
| | 90 | 77.9 | 3605 |
| | 120 | 90.8 | 6135 |
| | 180 | 114.5 | 12293 |
| | 240 | 132.8 | 19711 |
| | 360 | 165.8 | 37627 |
| | 525 | 196.0 | 67473 |
| | 720 | 212.3 | 107280 |
| Control B; | 0 | 0.0 | 0 |
| Crystalline drug | 4 | 90.1 | 180 |
| alone | 10 | 101.0 | 754 |
| | 20 | 98.8 | 1753 |
| | 40 | 166.1 | 4402 |
| | 90 | 147.9 | 12252 |
| | 120 | 164.5 | 16937 |
| | 180 | 137.3 | 25991 |
| | 240 | 141.8 | 34362 |
| | 360 | 161.0 | 52528 |
| | 525 | 179.0 | 80574 |
| | 720 | 157.2 | 113346 |
| Control C | 0 | 0.0 | 0 |
| SD alone | 4 | 0.0 | 0 |
| | 10 | 44.1 | 1324 |
| | 20 | 468.0 | 5870 |
| | 40 | 479.0 | 15338 |
| | 90 | 505.5 | 39945 |
| | 120 | 492.6 | 54916 |
| | 180 | 536.6 | 85792 |
| | 240 | 531.8 | 117840 |
| | 360 | 556.8 | 183153 |
| | 525 | 550.7 | 274517 |
| | 720 | 523.1 | 379210 |

| | | | |
|---|---|---|---|
| * µg/mL **min•µg/mL | | | |

### EXAMPLE 2

Exemplary dosage forms of the present invention were fabricated by first forming a batch of 2:1 SD comprising 2 parts of a low solubility drug to 1 part of dispersion polymer by mixing HPMCAS-MF with the drug [R-(R*S*)]-5 chloro-N-[2-hydroxy-3-[(methoxymethylamino)-3-oxo-1(phenylmethyl)propyl]propyl]-1H-indole-2-carboxymide, (another glyocogen phosphorylase inhibitor from Pfizer, Inc.) having a water solubility of 1 µg/mL. The solid dispersion was made as described in Example 1 except that the makeup of the solution was 5.0 wt% drug, 2.5 wt% HPMCAS-MF and 92.5 wt% solvent and the solution was spray-dried using a rotary atomizer nozzle set at 7.5 bar with an inlet temperature of 120°C. The drug dispersion was verified to be in an amorphous, non-crystalline state.

To incorporate the resulting SD into an erodible matrix, 1.05 g of the resulting solid particles were then mixed with 1.7 g METHOCEL K100LV Prem, 0.70 g of the filler FAST FLOW lactose, and 0.0525 g of the lubricant magnesium stearate, then tableted as described in Example 1. The resulting tablets were 20 wt% drug, 10 wt% HPMCAS-MF, 48.5 wt% METHOCEL, 20 wt% lactose and 1.5 wt% magnesium stearate. The tablet weight was 350 mg.

As a control (Control D), tablets were prepared similarly except rather than using the drug/HPMCAS-MF SD, a mixture of 2 parts crystalline drug to 1 part HPMCAS-MF was used. As a second control (Control E), 70 mg of the undispersed crystalline drug with no erodible matrix was used. As a third control (Control F), 107 mg of the SD with no erodible matrix was used.

A Model Fasted Duodenum (MFD) solution which mimics the chemical environment in the small intestine was prepared, comprising the PBS solution described in Example 1 mixed with 14.7 mM sodium taurocholic acid and 2.8 mM of 1-palmitoyl-2-oleyl-sn-glycero-3-phosphocholine. Four *in vitro* dissolution tests were performed in 500 mL of the so-prepared MFD solution to test the effectiveness of drug release from the dosage composition of the invention against Controls D, E and F. The sampling and analysis were carried out as in Example 1.

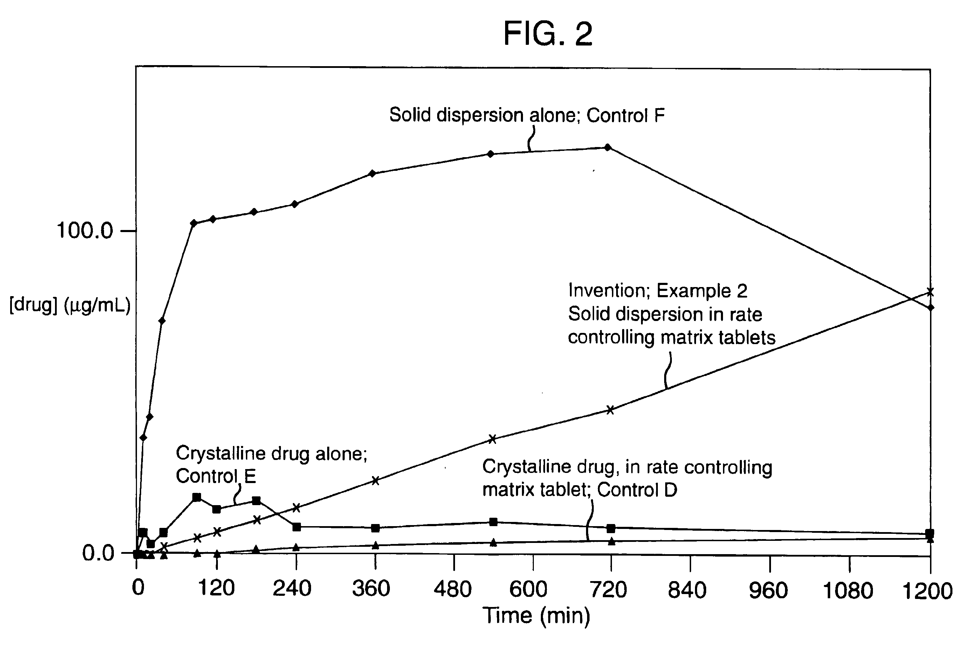

The results are set forth in Table 2 and graphically illustrated in Figure 2. As is apparent from Table 2 and Figure 2 the dosage form of the present invention exhibited a gradual and continuous increase in drug concentration in the MFD solution over a period of 20 hours, reaching a maximum value of about 82 µg/mL, 12 times that achieved by Control D and 4.6 times that achieved by Control E.

**Table 2**

| Dosage Form | Time (min) | [Drug]* | AUC** |
|---|---|---|---|
| Example 2 of | 0 | 0.0 | 0 |
| invention; | 10 | 0.0 | 0 |
| SD in erodible | 20 | 0.0 | 0 |
| matrix | 40 | 2.3 | 23 |
| | 90 | 5.2 | 212 |
| | 120 | 7.1 | 397 |
| | 180 | 10.9 | 936 |
| | 240 | 14.8 | 1706 |
| | 360 | 23.6 | 4011 |
| | 525 | 6.9 | 9458 |
| | 720 | 46.2 | 16939 |
| | 1200 | 82.4 | |
| Control D; | 0 | 0.0 | 0 |
| Crystalline drug | 10 | 0.0 | 0 |
| mixed with | 20 | 0.0 | 0 |
| dispersion polymer | 40 | 0.0 | 0 |
| | 90 | 0.0 | 0 |
| | 120 | 0.0 | 47 |
| | 180 | 1.6 | 154 |
| | 240 | 2.0 | 462 |
| | 360 | 3.1 | 1135 |
| | 525 | 4.4 | 1985 |
| | 720 | 5.1 | 2439 |
| | 1200 | 6.6 | |
| Control E; | 0 | 0.0 | 0 |
| Crystalline drug | 10 | 6.9 | 21 |
| alone | 20 | 3.2 | 72 |
| | 40 | 6.8 | 172 |
| | 90 | 18.1 | 795 |
| | 120 | 14.3 | 1281 |
| | 180 | 17.0 | 2219 |
| | 240 | 8.8 | 2992 |
| | 360 | 8.5 | 4030 |
| | 525 | 10.7 | 5756 |
| | 720 | 9.0 | 7525 |
| | 1200 | 8.0 | |
| | 0 | 0.0 | 0 |
| Control F; | 10 | 37.0 | 0 |
| SD alone | 20 | 43.6 | 111 |
| | 40 | 72.5 | 514 |
| | 90 | 102.4 | 1674 |
| | 120 | 103.6 | 6046 |
| | 180 | 105.8 | 9136 |
| | 240 | 108.2 | 15418 |
| | 360 | 117.8 | |
| | 525 | 124.0 | 21838 |
| | 720 | 126.3 | 35401 |
| | 1220 | 78.0 | 57168 |

| | | | |
|---|---|---|---|
| * µg/mL **min•µg/mL | | | |

This Example also demonstrates that the dosage form of the present invention is capable of a much more desirable tₘₐₓ as compared to that of an SD alone, reaching its maximum concentration in at least 20 hours, whereas drug released from the SD alone (Control F) reached a plateau concentration near its maximum concentration in less than 90 minutes. The matrix tablets were completely disintegrated over the course of the 20 hours, indicating that the mechanism of controlled release was primarily by erosion.

### REFERENCE EXAMPLE 3

Exemplary dosage forms of the present invention were fabricated by first forming a batch of 1:3 SD of 1 part of a low solubility drug to 3 parts of the dispersion polymer by mixing the same drug as in Example 1 with CAP to form a solution. The spray-drying was carried out as described in Example 1 except that the solution was 0.75 wt% drug, 2.25 wt% CAP and 97 wt% acetone and the spray apparatus was set at 1.9 bar. Formation of an amorphous, noncrystalline drug dispersion was confirmed.

To incorporate the solid dispersion into an erodible matrix, 1.071 g of the resulting solid particles were then mixed with 1.7315 g METHOCEL, 0.714 g of lactose filler, and 0.0536 g of the lubricant magnesium stearate. The tablet weight was 350 mg.

As a control (Control G), tablets were similarly prepared except rather than using the drug/CAP solid dispersion, a mixture of 1 part crystalline drug to 3 parts CAP was used. As a second control (Control H), 26 mg of crystalline drug alone was used. As a third control (Control I), 105.3 mg of the solid dispersion with no erodible matrix was used. In order to determine the drug release profile of the dosage form of the present invention as compared to the controls, in vitro dissolution tests in 40 mL of the PBS solution of Example 1 at 37°C were performed as in Example 1.

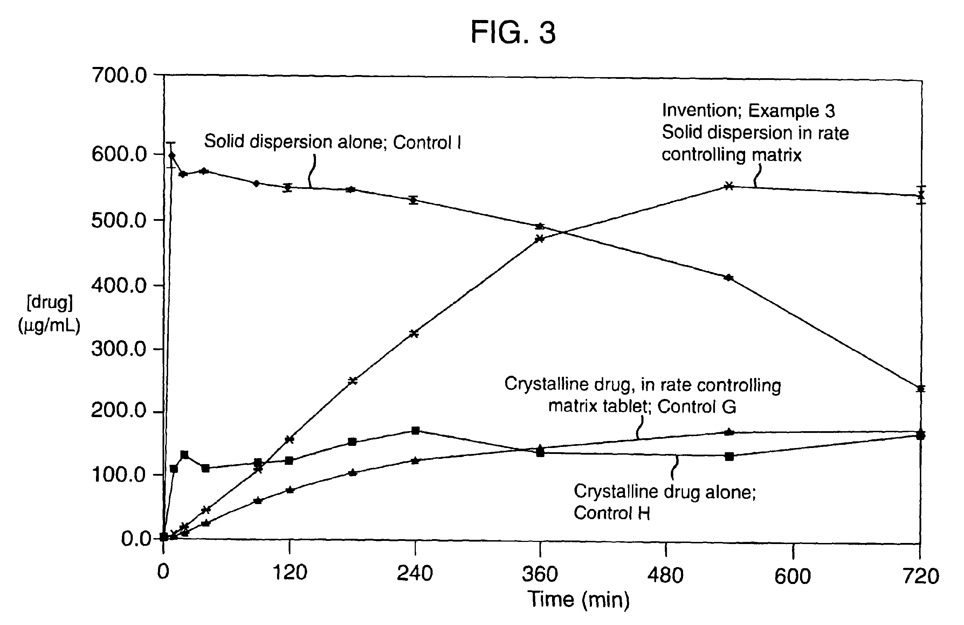

The results are set forth in Table 3 and graphically illustrated in FIG. 3, and as is apparent therefrom, the dosage form of the present invention exhibited a gradual and continuous increase in drug concentration in the MFD solution over a period of about 9 hours, reaching a maximum value of about 560 µg/mL-more than three-fold greater than that achieved by either Control G or Control H. This Example further demonstrates that the dosage form of the present invention has a much more desirable tₘₐₓ than does the SD alone, reaching its maximum drug concentration in about 9 hours, whereas maximum concentration for the SD alone (Control I) is reached in less than 10 minutes. Finally, the data obtained show that supersaturated drug concentrations are attainable, more than three-fold that achieved by the crystalline drug alone (Control H) or the crystalline drug mixed with CAP (Control G). The matrix tablets were completely disintegrated over the course of the 12 hours, indicating that the mechanism of controlled release was primarily by erosion.

**Table 3**

| Dosage Form | Time (min) | [Drug]* | AUC** |
|---|---|---|---|
| Example 4 of | 0 | 0.0 | 0 |
| invention; | 10 | 6.8 | 21 |
| SD in erodible | 20 | 18.8 | 149 |
| matrix | 40 | 46.3 | 799 |
| | 90 | 107.9 | 4654 |
| | 120 | 157.8 | 8640 |
| | 180 | 248.4 | 20826 |
| | 240 | 328.5 | 38131 |
| | 360 | 474.7 | 86322 |
| | 525 | 557.0 | 171442 |
| | 720 | 545.3 | 278918 |
| Control G; | 0 | 0.0 | 0 |
| Crystalline drug | 10 | 2.9 | 9 |
| mixed with | 20 | 9.7 | 72 |
| dispersion polymer | 40 | 25.4 | 423 |
| | 90 | 60.7 | 2575 |
| | 120 | 76.7 | 4636 |
| | 180 | 105.2 | 10091 |
| | 240 | 125.2 | 17003 |
| | 360 | 146.1 | 33282 |
| | 540 | 174.2 | 59702 |
| | 720 | 177.5 | 93992 |
| Control H; | 0 | 0.0 | 0 |
| Crystalline drug | 10 | 107.8 | 0 |
| alone | 20 | 130.1 | 323 |
| | 40 | 109.1 | 1513 |
| | 90 | 118.1 | 3906 |
| | 120 | 121.8 | 9587 |
| | 180 | 151.7 | 13186 |
| | 240 | 170.4 | 21392 |
| | 360 | 135.3 | 31055 |
| | 540 | 134.8 | 49395 |
| | 720 | 168.3 | 73701 |
| Control I; | 0 | 0.0 | 0 |
| SD alone | 10 | 589.7 | 0 |
| | 20 | 560.6 | 1769 |
| | 40 | 566.4 | 7520 |
| | 90 | 549.2 | 18790 |
| | 120 | 543.1 | 46680 |
| | 180 | 540.3 | 63064 |
| | 240 | 526.2 | 95564 |
| | 360 | 487.6 | 127556 |
| | 525 | 412.6 | 188381 |
| | 720 | 242.0 | 262646 |

| | | | |
|---|---|---|---|
| * µg/mL **min•µg/mL | | | |

### EXAMPLE 4

Tablets of a solid drug dispersion incorporated into an erodible matrix were prepared as in Example 2, then coated with a 100 Å-thick enteric coating to delay drug release, comprising 90 wt% CAP and 10 wt% triacetin. The coating was applied by spraying a solution comprising 9 wt% CAP and 1 wt% triacetin in acetone onto the tablet cores using a conventional pan coater. The drug release profile of this coated dosage form was tested by exposing the tablets to USP simulated gastric buffer for two hours and then transferring the tablets to the MFD solution of Example 2. Release of drug was delayed by two to four hours, showing the effectiveness of the coating for retarding drug release while the tablets are in the gastric environment.

### REFERENCE EXAMPLE 5

Exemplary dosage forms of the present invention were fabricated as in Example 1 by first forming a batch of SD comprising 1 part of the low solubility drug (1S-*cis*)-4-(3,4-dichlorophenyl)-1,2,3,4-tetrahydro-N-methyl-1-napthalenamine hydrochloride having a water solubility of 70 µg/mL and 1 part of the dispersion polymer HPMCP-55 except that drug and polymer were sprayed from a 1:1 mixture (by weight) of MeOH:acetone with a solution composition of 2.5 wt% drug, 2.5 wt% polymer and 92.5 wt% solvent. The solution was sprayed at a pressure of 1.8 bar with a 190 g/min feed rate, a 230°C inlet temperature, and a 70°C outlet temperature. The drug dispersion was verified to be in an amorphous, non-crystalline state.

To incorporate the resulting SD into an erodible matrix, a homogeneous mixture of 30% SD, 48.5% METHOCEL K100LV Prem, 20% FAST FLO lactose and 1.5% magnesium stearate was made. Tablets were made as described in Example 1.

As a control (Control J), tablets were prepared similarly except rather than using the drug/HMPCP SD, a mixture of 1 part crystalline drug to 1 part of the same grade of HPMCP was used. As a second control (Control K), 62 mg of the undispersed crystalline drug alone was used. As a third control (Control L), 122 mg of the SD with no erodible matrix was used.

An MFD solution was prepared as in Example 2. Four *in vitro* dissolution tests were performed in 30 ml of the MFD solution to test the effectiveness of drug release from the dosage composition of the invention against Controls J, K and L. The sampling and analysis were carried out as in Example 1.

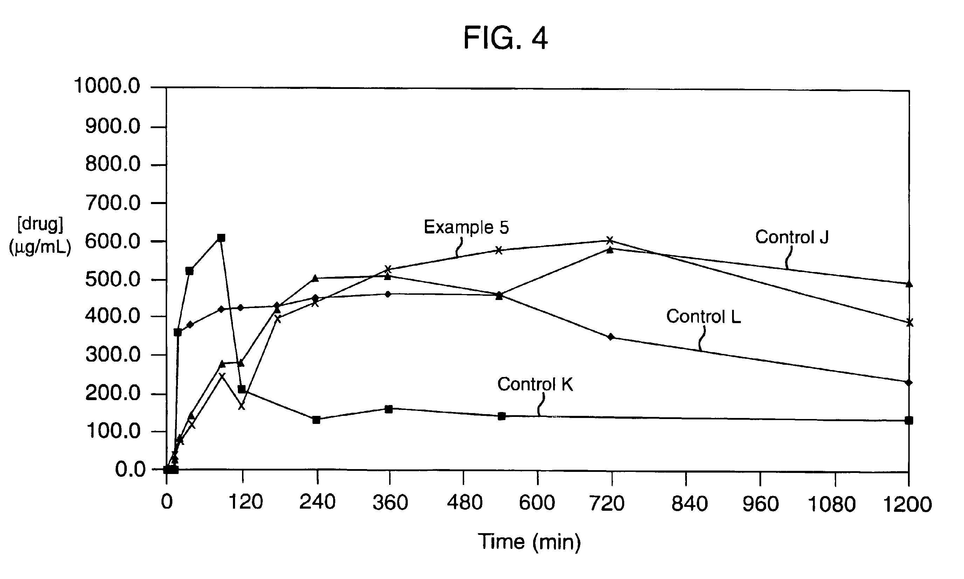

The results are set forth in Table 4 and graphically illustrated in FIG. 4, and as is apparent therefrom the dosage form of the present invention exhibited a gradual and continuous increase in drug concentration in the MFD solution over a period of 12 hours, reaching its maximum value of about 600 µg/mL at 12 hours. At 9 hours the drug concentratin achieved by the dosage composition of the invention was 4-fold that of Control K.

**Table 4**

| Dosage Form | Time (min) | [Drug] * | AUC** |
|---|---|---|---|
| Example 6 of | 0 | 0 | 0 |
| invention; SD | 10 | 35 | 104 |
| in erodible | 20 | 74 | 650 |
| matrix | 40 | 118 | 2570 |
| | 90 | 245 | 11629 |
| | 120 | 166 | 17785 |
| | 180 | 395 | 34613 |
| | 240 | 436 | 59546 |
| | 360 | 523 | 117090 |
| | 540 | 573 | 215775 |
| | 720 | 600 | 321320 |
| | 1200 | 380 | |
| Control J; | 0 | 0 | 0 |
| Crystalline | 10 | 27 | 82 |
| drug mixed with | 20 | 88 | 656 |
| dispersion | 40 | 145 | 2981 |
| polymer | 90 | 277 | 13522 |
| | 120 | 283 | 21922 |
| | 180 | 421 | 43043 |
| | 240 | 503 | 70742 |
| | 360 | 506 | 131280 |
| | 540 | 454 | 217682 |
| | 720 | 579 | 310606 |
| | 1200 | 485 | |
| Control K; | 0 | 0 | 0 |
| Crystalline | 10 | 0 | 1073 |
| drug alone | 20 | 358 | 5459 |
| | 40 | 520 | 16706 |
| | 90 | 605 | 37083 |
| | 120 | 210 | 40231 |
| | 240 | 130 | 52796 |
| | 360 | 159 | 70528 |
| | 540 | 136 | 108647 |
| | 1200 | 128 | |
| Control L; | 0 | 0 | 0 |
| SD alone | 10 | 0 | 1076 |
| | 20 | 359 | 4751 |
| | 40 | 376 | 12704 |
| | 90 | 419 | 33693 |
| | 120 | 421 | 46399 |
| | 180 | 426 | 72602 |
| | 240 | 447 | 99737 |
| | 360 | 457 | 154514 |
| | 540 | 456 | 226262 |
| | 720 | 342 | 277001 |
| | 1200 | 222 | |

| | | | |
|---|---|---|---|
| * µg/mL **min•µg/mL | | | |

## Claims

1. A controlled release dosage composition comprising:
(a) a solid, substantially homogeneous, dispersion comprising a low solubility drug dispersed in hydroxypropylmethylcellulose acetate succinate cellulosic polymer, a major portion of said drug being amorphous; and
(b) an aqueous-soluble cellulosic polymeric matrix having said dispersion incorporated therein;
wherein the dispersion is formed by spray drying;
wherein a substantially homogenous dispersion is one in which the dispersion itself has a single glass transition temperature (T_{g});
wherein the low solubility drug is one which has a minimum aqueous solubility at physiologically relevant pH 1-8 of 40mg/ml or less; and
wherein a major portion of said drug being amorphous means that at least 60% of the drug in the dispersion is In the amorphous form.

2. The dosage composition of Claim 1 wherein said aqueous-soluble cellulosic polymer is selected from the group consisting of methylcellulose, hydroxyethylcellulose, hydroxypropylcellulose, hydroxyethylmethylcellulose and hydroxypropylmethylcellulose.

3. The dosage composition of Claim 1 wherein substantially all of said drug is substantially amorphous wherein substantially all of said drug is in the amorphous form means that at least 75% of the drug in the dispersion is in the amorphous form.

4. The dosage composition of Claim 1 wherein essentially all of said drug is substantially amorphous wherein essentially all of said drug is amorphous means that at least 90% of the drug in the dispersion is in the amorphous form.

5. The dosage composition of Claim 1 wherein said polymeric matrix is coated with a pH-sensitive polymer to delay the release of said drug.

6. The dosage composition of Claim 5 wherein said pH-sensitive polymer is selected from the group consisting of amylase acetate phthalate, cellulose acetate phthalate and its sodium salt, cellulose ester phthalates, cellulose ether phthalates, hydroxypropylcellulose phthalate, hydroxypropylethylcellulose phthalate, hydroxypropylmethylcellulose phthalate, hydroxypropylmethylcellulose acetate succinate, methylcellulose phthalate, polyvinyl acetate phthalate, polyvinyl acetate hydrogen phthalate, starch acid phthalate, cellulose acetate trimellitate, styrene-maleic acid dibutyl phthalate copolymer, styrene-maleic acid/polyvinylacetate phthalate copolymer, styrene and maleic acid copolymers, acrylic acid and acrylic ester copolymers, polymethacrylic acid and esters thereof, polyacrylic and methacrylic acid copolymers, shellac, and vinyl acetate and crotonic acid copolymers.

7. The dosage composition of Claim 5 wherein said pH sensitive polymer is selected from the group consisting of cellulose acetate phthalate, polyvinylacetate phthalate, hydroxypropylmethylcellulose phthalate, hydroxypropylmethylcellulose acetate succinate, anionic acrylic copolymers of methacrylic acid and methylmethacrylate, and copolymers comprising acrylic acid and at least one acrylic acid ester.

8. The dosage composition of Claim 5 wherein said coating further comprises at least one non-pH-sensitive polymer to modulate either the delay of release or the rate of release of said drug.

9. The dosage composition of Claim 1 in a form selected from the group consisting of a tablet, a caplet, a capsule, a bead, a sachet, a multiparticulate and combinations thereof.

10. The dosage composition of Claim 1 wherein said dispersion includes a solubility enhancing agent.

11. The dosage composition of Claim 10 wherein said solubility enhancing agent is selected from a group consisting of organic acids and organic salts; partial glycerides; glycerides; glyceride derivatives; polyethylene glycol esters; polypropylene glycol esters; polyhydric alcohol esters; sorbitan esters; carbonate salts; alkyl sulfonates; and cyclodextrins.

12. The dosage composition of Claim 1 wherein, prior to its disperslon, said drug in its pure state is amorphous.

13. The dosage composition of Claim 1 wherein, prior to its dispersion, said drug in its pure state is crystalline.

14. The dosage composition of Claim 1 wherein said dispersion includes excipients.

15. The dosage composition of Claim 14 wherein said excipients are selected from the group consisting of surfactants, aqueous soluble polymers, pH modifiers, fillers, binders, pigments, lubricants, antioxidants and flavorants.

16. The dosage composition of Claim 1 wherein the drug is selected from the group consisting of an anti-hypertensive, an antianxiety agent, an anticlotting agent, a blood glucose-lowering agent, a decongestant, an antihistamine, an antitussive, an anti-inflammatory, an antipsychotic agent, a cognitive enhancer, a cholesterol-reducing agent, an antiobesity agent, an autoimmune disorders agent, a hypnotic agent, an anti-Parkinsonism agent, an antibiotic agent, an antiviral agent, an anti-impotence agent, an anti-neoplastic, a sedative, a barbiturate, a nutritional agent, a beta-blocker, an emetic, an anti-emetic, a diuretic, an anticoagulant, a cardiotonic, an androgen, a corticoid, anabolic agent, an anti-depression agent, an anti-infective agent, a coronary vasodilator, a carbonic anhydrase inhibitor, an antifungal, an antiprotozoal, a gastrointestinal agent, a dopaminergic agent, an anti-Alzheimer's Disease agent, an anti-ulcer agent, a platelet inhibitor, and a glycogen phosphorylase inhibitor.

17. The dosage composition of Claim 16 wherein said drug is an antihypertensitive selected from the group consisting of prazosin, nifedipine, trimazosin and doxazosin.

18. The dosage composition of Claim 16 wherein said drug is an antianxiety agent selected from the group consisting of fluoxetine, pyroxidine, sertraline, venlafaxine, [3, 6-dimethyl-2-(2, 3, 6-trimethylphenoxy)-pyridi-4-yl]-(1-ethylpropyl)-amine and 3, 5-dimethyl-4(3'-pentoxy)-2-(2', 4',6'-trimethylphenoxy)pyridine.

19. The dosage composition of Claim 16 wherein said drug is the antipsychotic agent selected from the group consisting of ziprasidone and its pharmaceutically acceptable salts.

20. The dosage composition of Claim 16 wherein said drug is the anti-impotence agent selected form the group consisting of sildenafil and its pharmaceutically acceptable salts.

21. The dosage composition of Claim 16 wherein said drug is the blood glucose-lowering agent glipizide.

22. The dosage composition of Claim 16 wherein said drug is a glycogen phosphorylate inhibitor selected from the group consisting of [R-(R*, S*)]-5-chloro-N-[2-hydroxy-3-[methyoxymethylamino)-3-oxo-1-(phenylmethyl) propyl) propyl]-1H-indole-2-carboxamide and 5-chloro-1H-indole-2-carboxylic acid [(1S)-benzyl-3-(3R,4S)-dihyroxypyrrolidin-1-yl-)-(2R)-hydroxy-3-oxypropyl]amide.

23. The dosage composition of Claim 16 wherein said drug is a anti-inflammatory selected from the group consisting of iroxicam, valdecoxib and celicoxib.

24. The dosage composition of Claim 23 wherein said drug is celicoxib.

25. The use of a controlled release dosage composition of Claim 1 for the preparation of a medicament for treating a disease or disorder in a mammal.

## Patentansprüche

1. Dosierungszusammensetzung mit kontrollierter Freisetzung, umfassend:
(a) eine feste, im Wesentlichen homogene, Dispersion, umfassend einen Wirkstoff mit geringer Löslichkeit, dispergiert in Hydroxypropylmethylcelluloseacetatsuccinat-Cellulosepolymer, wobei ein Hauptteil des Wirkstoffs amorph ist; und
(b) eine wasserlösliche Cellulosepolymermatrix, die die Dispersion darin eingearbeitet aufweist;
wobei die Dispersion durch Sprühtrocknen gebildet wird;
wobei eine im Wesentlichen homogene Dispersion eine ist, in welcher die Dispersion selbst eine einzelne Glasübergangstemperatur (Tg) aufweist;
wobei der Wirkstoff mit geringer Löslichkeit einer ist, der eine minimale Löslichkeit in Wasser bei physiologisch relevantem pH von 1-8 von 40 mg/ml oder weniger aufweist; und
wobei ein Hauptteil des Wirkstoffs ist amorph bedeutet, dass mindestens 60% des Wirkstoffs in der Dispersion in amorpher Form vorliegen.

2. Dosierungszusammensetzung nach Anspruch 1, wobei das wasserlösliche Cellulosepolymer ausgewählt ist aus der Gruppe, bestehend aus Methylcellulose, Hydroxyethylcellulose, Hydroxypropylcellulose, Hydroxyethylmethylcellulose und Hydroxypropylmethylcellulose.

3. Dosierungszusammensetzung nach Anspruch 1, wobei im Wesentlichen der gesamte Wirkstoff im Wesentlichen amorph ist, wobei im Wesentlichen der gesamte Wirkstoff liegt in der amorphen Form vor bedeutet, das mindestens 75% des Wirkstoffs in der Dispersion in der amorphen Form vorliegen.

4. Dosierungszusammensetzung nach Anspruch 1, wobei im Wesentlichen der gesamte Wirkstoff im Wesentlichen amorph ist, wobei im Wesentlichen der gesamte Wirkstoff ist amorph bedeutet, dass mindestens 90% des Wirkstoffs in der Dispersion in der amorphen Form vorliegen.

5. Dosierungszusammensetzung nach Anspruch 1, wobei die Polymermatrix mit einem pH-sensitiven Polymer beschichtet ist, um die Freisetzung des Wirkstoffs zu verzögern.

6. Dosierungszusammensetzung nach Anspruch 5, wobei das pH-sensitive Polymer ausgewählt ist aus der Gruppe, bestehend aus Amyloseacetatphthalat, Celluloseacetatphthalat und seinem Natriumsalz, Celluloseesterphthalaten, Celluloseetherphthalaten, Hydroxypropylcellulosephthalat, Hydroxypropylethylcellulosephthalat, Hydroxypropylmethylcellulosephthalat, Hydroxypropylmethylcelluloseacetatsuccinat, Methylcellulosephthalat, Polyvinylacetatphthalat, Polyvinylacetathydrogenphthalat, Stärkesäurephthalat, Celluloseacetattrimellitat, Styrol-Maleinsäuredibutylphthalat-Copolymer, Styrol-Maleinsäure/Polyvinylacetatphthalat-Copolymer, Copolymeren von Styrol und Maleinsäure, Copolymeren von Acrylsäure und Acrylestern, Polymethacrylsäure und Estern davon, Polyacryl- und -methacrylsäure-Copolymeren, Schellack und Copolymeren von Vinylacetat und Crotonsäure.

7. Dosierungszusammensetzung nach Anspruch 5, wobei das pH-sensitive Polymer ausgewählt ist aus der Gruppe, bestehend aus Celluloseacetatphthalat, Polyvinylacetatphthalat, Hydroxypropylmethylcellulosephthalat, Hydroxypropylmethylcelluloseacetatsuccinat, anionischen Acrylcopolymeren von Methacrylsäure und Methylmethacrylat und Copolymeren, umfassend Acrylsäure und mindestens einen Acrylsäureester.

8. Dosierungszusammensetzung nach Anspruch 5, wobei die Beschichtung weiterhin mindestens ein nicht-pH-sensitives Polymer umfasst, um entweder die Verzögerung der Freisetzung oder die Freisetzungsrate des Wirkstoffs zu modulieren.

9. Dosierungszusammensetzung nach Anspruch 1 in einer Form, ausgewählt aus der Gruppe, bestehend aus einer Tablette, einem Caplet, einer Kapsel, einem Kügelchen, einem Sachet, einem Multipartikulat und von Kombinationen davon.

10. Dosierungszusammensetzung nach Anspruch 1, wobei die Dispersion ein die Löslichkeit erhöhendes Mittel einschließt.

11. Dosierungszusammensetzung nach Anspruch 10, wobei das die Löslichkeit erhöhende Mittel ausgewählt ist aus einer Gruppe, bestehend aus organischen Säuren und organischen Salzen, partiellen Glyceriden, Glyceriden, Glyceridderivaten, Polyethylenglykolestern, Polypropylenglykolestern, Polyolestern, Sorbitanestern, Carbonatsalzen, Alkylsulfonaten und Cyclodextrinen.

12. Dosierungszusammensetzung nach Anspruch 1, wobei der Wirkstoff, vor seiner Dispersion, in seinem reinen Zustand amorph ist.

13. Dosierungszusammensetzung nach Anspruch 1, wobei der Wirkstoff, vor seiner Dispersion, in seinem reinen Zustand kristallin ist.

14. Dosierungszusammensetzung nach Anspruch 1, wobei die Dispersion Exzipientien einschließt.

15. Dosierungszusammensetzung nach Anspruch 14, wobei die Exzipientien ausgewählt sind aus der Gruppe, bestehend aus oberflächenaktiven Mitteln, wasserlöslichen Polymeren, pH-Modifikatoren, Füllstoffen, Bindemitteln, Pigmenten, Schmier- bzw. Gleitmitteln, Antioxidantien und aromagebenden Mitteln.

16. Dosierungszusammensetzung nach Anspruch 1, wobei der Wirkstoff ausgewählt ist aus der Gruppe, bestehend aus einem Antihypertonikum, einem Anxiolytikum, einem gerinnungshemmenden Mittel, einem Blutglucosesenkungsmittel, einem Entstauungsmittel, einem Antihistaminikum, einem Antitussivum, einem entzündungshemmenden Mittel, einem Antipsychotikum, einem Mittel zur Verbesserung der kognitiven Fähigkeit bzw. cognitiven Enhancer, einem cholesterinsenkenden Mittel, einem Anorektikum, einem Mittel bei Autoimmunstörungen, einem Hypnotikum, einem Anti-Parkinson-Mittel, einem Antibiotikum, einem antiviralen Mittel, einem Antiimpotenzmittel, einem Antineoplastikum, einem Sedativum, einem Barbiturat, einem Mittel zur Ernährung, einem Betablocker, einem Emetikum, einem Antiemetikum, einem Diuretikum, einem Antikoagulans, einem Kardiotonikum, einem Androgen, einem Kortikoid, einem Anabolikum, einem Antidepressivum, einem Antiinfektivum, einem Koronarvasodilatans, einem Carboanhydraseinhibitor, einem Antimykotikum, einem Antiprotozoenmittel, einem Gastrointestinalmittel, einem dopaminergen Mittel, einem Mittel gegen Alzheimer-Krankheit, einem Antiulzerativum, einem Thrombozyteninhibitor und einem Glycogenphosporylaseinhibitor.

17. Dosierungszusammensetzung nach Anspruch 16, wobei der Wirkstoff ein Antihypertonikum ist, ausgewählt aus der Gruppe, bestehend aus Prazosin, Nifedipin, Trimazosin und Doxazosin.

18. Dosierungszusammensetzung nach Anspruch 16, wobei der Wirkstoff ein Anxiolytikum ist, ausgewählt aus der Gruppe, bestehend aus Fluoxetin, Pyroxidin, Sertralin, Venlafaxin, [3,6-Dimethyl-2-(2,3,6-trimethylphenoxy)pyridin-4-yl]-(1-ethylpropyl)amin und 3,5-Dimethyl-4-(3'-pentoxy)-2-(2',4',6'-trimethylphenoxy)pyridin.

19. Dosierungszusammensetzung nach Anspruch 16, wobei der Wirkstoff das Antipsychotikum ist, ausgewählt aus der Gruppe, bestehend aus Ziprasidon und seinen pharmazeutisch verträglichen Salzen.

20. Dosierungszusammensetzung nach Anspruch 16, wobei der Wirkstoff das Antiimpotenzmittel ist, ausgewählt aus der Gruppe, bestehend aus Sildenafil und seinen pharmazeutisch verträglichen Salzen.

21. Dosierungszusammensetzung nach Anspruch 16, wobei der Wirkstoff das Blutglucosesenkungsmittel'Glipizid ist.

22. Dosierungszusammensetzung nach Anspruch 16, wobei der Wirkstoff ein Glycogenphosphorylaseinhibitor ist, ausgewählt aus der Gruppe, bestehend aus [R-(R*,S*)]-5-Chlor-N-[2-hydroxy-3-[methoxymethylamino)-3-oxo-1-(phenylmethyl)propyl)propyl]-1H-indol-2-carboxamid und 5-Chlor-1H-indol-2-carbonsäure-[(1S)-benzyl-3-(3R,4S)-dihydroxypyrrolidin-1-yl)-(2R)-hydroxy-3-oxypropyl]amid.

23. Dosierungszusammensetzung nach Anspruch 16, wobei der Wirkstoff ein entzündungshemmendes Mittel ist, ausgewählt aus der Gruppe, bestehend aus Iroxicam, Valdecoxib und Celicoxib.

24. Dosierungszusammensetzung nach Anspruch 23, wobei der Wirkstoff Celicoxib ist.

25. Verwendung einer Dosierungszusammensetzung mit kontrollierter Freisetzung nach Anspruch 1 für die Herstellung eines Medikaments zur Behandlung einer Krankheit oder Störung bei einem Säuger.

## Revendications

1. Composition dosée à libération contrôlée, comprenant :
(a) une dispersion solide, sensiblement homogène, comprenant un médicament de faible solubilité, dispersé dans un polymère cellulosique constitué de succinate d'acétate d'hydroxypropylméthylcellulose, la majeure partie dudit médicament étant amorphe ; et
(b) une matrice polymère cellulosique hydrosoluble incorporant, en son sein, ladite dispersion ;
où :
la dispersion est formée par séchage par pulvérisation ;
une dispersion sensiblement homogène étant une dispersion dans laquelle la dispersion en elle-même a une température de transition vitreuse unique (T_{g}) ;
le médicament de faible solubilité étant un médicament qui a une solubilité aqueuse minimale à un pH physiologiquement approprié, compris entre 1 et 8, de 40 mg/ml ou moins ; et
"la majeure partie dudit médicament étant amorphe" signifiant que 60 % au moins du médicament, dans la dispersion, est sous forme amorphe.

2. Composition dosée selon la revendication 1, dans laquelle ledit polymère cellulosique hydrosoluble est sélectionné dans le groupe consistant en la méthylcellulose, l'hydroxyéthylcellulose, l'hydroxypropylcellulose, l'hydroxyéthylméthylcellulose et l'hydroxypropylméthylcellulose.

3. Composition dosée selon la revendication 1, dans laquelle sensiblement la totalité dudit médicament est sensiblement amorphe, où "sensiblement la totalité dudit médicament étant sous forme amorphe" signifie que 75 % au moins du médicament dans la dispersion est sous forme amorphe.

4. Composition dosée selon la revendication 1, dans laquelle essentiellement la totalité dudit médicament est sensiblement amorphe, où "essentiellement la totalité dudit médicament étant amorphe" signifie que 90 % au moins du médicament dans la dispersion est sous forme amorphe.

5. Composition dosée selon la revendication 1, dans laquelle ladite matrice polymère est enrobée d'un polymère pH-sensible pour différer la libération dudit médicament.

6. Composition dosée selon la revendication 5, dans laquelle ledit polymère pH-sensible est sélectionné dans le groupe consistant en le phtalate d'acétate d'amylase, le phtalate d'acétate de cellulose et son sel sodique, les phtalates d'ester de cellulose, les phtalates d'éther de cellulose, le phtalate d'hydroxypropylcellulose, le phtalate d'hydroxypropyléthylcellulose, le phtalate d'hydroxypropylméthylcellulose, le succinate d'acétate d'hydroxypropylméthylcellulose, le phtalate de méthylcellulose, le phtalate de poly(acétate de vinyle), l'hydrogénophtalate de poly(acétate de vinyle), le phtalate acide d'amidon, le trimellitate d'acétate de cellulose, le copolymère styrène-acide maléique-phtalate de dibutyle, le copolymère styrène-acide maléique/phtalate de poly(acétate de vinyle), les copolymères de styrène et d'acide maléique, les copolymères d'acide acrylique et d'ester acrylique, les acides et esters polyméthacryliques correspondants, les copolymères de poly(acide acrylique et méthacrylique), le shellac et les copolymères d'acétate de vinyle et d'acide crotonique.

7. Composition dosée selon la revendication 5, dans laquelle ledit polymère pH-sensible est sélectionné dans le groupe consistant en le phtalate d'acétate de cellulose, le phtalate de poly(acétate de vinyle), le phtalate d'hydroxypropylméthylcellulose, le succinate d'acétate d'hydroxypropylméthylcellulose, les copolymères acryliques anioniques d'acide méthacrylique et de méthacrylate de méthyle et les copolymères comprenant de l'acide acrylique et au moins un ester de l'acide acrylique.

8. Composition dosée selon la revendication 5, dans laquelle ledit enrobage comprend en outre au moins un polymère non-pH-sensible pour moduler soit le délai de libération, soit la vitesse de libération dudit médicament.

9. Composition dosée selon la revendication 1, sous une forme sélectionnée dans le groupe consistant en un comprimé, un caplet, une gélule, une bille, un sachet, une forme multiparticulaire, et les combinaisons de ceux-ci.

10. Composition dosée selon la revendication 1, dans laquelle ladite dispersion inclut un agent augmentant la solubilité.

11. Composition dosée selon la revendication 10, dans laquelle ledit agent augmentant la solubilité est sélectionné dans le groupe consistant en les acides organiques et les sels organiques ; les glycérides partiels ; les glycérides ; les dérivés de glycérides ; les esters de polyéthylèneglycol ; les esters de polypropylèneglycol ; les esters d'alcool polyhydrique ; les esters de sorbitane ; les sels de carbonate ; les alkylsulfonates ; et les cyclodextrines.

12. Composition dosée selon la revendication 1, dans laquelle, avant d'être dispersé, ledit médicament à l'état pur est amorphe.

13. Composition dosée selon la revendication 1, dans laquelle, avant d'être dispersé, ledit médicament à l'état pur est cristallin.

14. Composition dosée selon la revendication 1, dans laquelle ladite dispersion inclut des excipients.

15. Composition dosée selon la revendication 14, dans laquelle lesdits excipients sont sélectionnés dans le groupe consistant en les tensioactifs, les polymères hydrosolubles, les modificateurs de pH, les charges, les liants, les pigments, les lubrifiants, les antioxydants et les agents aromatisants.

16. Composition dosée selon la revendication 1, dans laquelle le médicament est sélectionné dans le groupe consistant en un antihypertenseur, un agent antianxiété, un agent anticoagulation, un agent diminuant le taux de glucose sanguin, un décongestionnant, un antihistaminique, un antitussif, un anti-inflammatoire, un agent antipsychotique, un psychostimulant, un agent réduisant le cholestérol, un agent antiobésité, un agent pour troubles auto-immuns, un agent hypnotique, un agent anti-parkinsonisme, un agent antibiotique, un agent antiviral, ùn agent anti-impuissance, un antinéoplasique, un sédatif, un barbiturique, un agent nutritionnel, un bêtabloquant, un émétique, un antiémétique, un diurétique, un anticoagulant, un cardiotonique, un androgène, un corticoïde, un agent anabolisant, un agent antidépression, un agent anti-infectieux, un vasodilatateur coronaire, un inhibiteur de l'anhydrase carbonique, un antifongique, un antiprotozoaire, un agent gastro-intestinal, un agent dopaminergique, un agent anti-maladie d'Alzheimer, un agent antiulcéreux, un antiagrégant plaquettaire et un inhibiteur de la glycogène phosphorylase.

17. Composition dosée selon la revendication 16, dans laquelle ledit médicament est un antihypertenseur sélectionné dans le groupe consistant en la prazosine, la nifédipine, la trimazosine et la doxazosine.

18. Composition dosée selon la revendication 16, dans laquelle ledit médicament est un agent antianxiété sélectionné dans le groupe consistant en la fluoxétine, la pyroxidine, la sertraline, la venlafaxine, la [3,6-diméthyl-2-(2,3,6-triméthylphénoxy)-pyridin-4-yl]-(1-éthylpropyl)-amine et la 3,5-diméthyl-4-(3'-pentoxy)-2-(2',4',6'-triméthylphénoxy)pyridine.

19. Composition dosée selon la revendication 16, dans laquelle ledit médicament est un agent antipsychotique sélectionné dans le groupe consistant en la ziprasidone et ses sels pharmaceutiquement acceptables.

20. Composition dosée selon la revendication 16, dans laquelle ledit médicament est un agent anti-impuissance sélectionné dans le groupe consistant en le sildénafil et ses sels pharmaceutiquement acceptables.

21. Composition dosée selon la revendication 16, dans laquelle ledit médicament est l'agent diminuant lé taux de glucose sanguin, nommé glipizide.

22. Composition dosée selon la revendication 16, dans laquelle ledit médicament est un inhibiteur de la glycogène phosphorylase sélectionné dans le groupe consistant en le [R-(R*,S*)]-5-chloro-N-[2-hydroxy-3-[méthyoxyméthylamino)-3-oxo-1-(phénylméthyl)propyl)propyl]-1H-indole-2-carboxamide et le [(1S)-benzyl-3-((3R,4S)-dihydroxypyrrolidin-1-yl)-(2R)-hydroxy-3-oxypropyl]amide de l'acide 5-chloro-1H-indole-2-carboxylique.

23. Composition dosée selon la revendication 16, dans laquelle ledit médicament est un anti-inflammatoire sélectionné dans le groupe consistant en le piroxicam, le valdécoxib et le célécoxib.

24. Composition dosée selon la revendication 23, dans laquelle ledit médicament est le célécoxib.

25. Utilisation d'une composition dosée à libération contrôlée selon la revendication 1 pour la préparation d'un médicament destiné à traiter une maladie ou un trouble chez un mammifère.
